# EUROPÄISCHE PATENTSCHRIFT

(11) **EP 2 876 163 B1**
(45) Veröffentlichungstag und Bekanntmachung des Hinweises auf die Patenterteilung: **10.01.2018**
(21) Anmeldenummer: 14194345.6
(22) Anmeldetag: 21.11.2014
(51) Int. Cl.: C12N 15/115, A61K 31/7088, A61P 35/00

(54) **DNA-Aptamere, die E- und P-Selektine spezifisch binden**
DNA aptamers specifically binding E- and P-Selectins
Aptamères d'ADN se liant spécifiquement E et P-sélectines

(30) Priorität: 22.11.2013 DE 102013112915
(43) Veröffentlichungstag der Anmeldung: 27.05.2015
(73) Patentinhaber: Universität Hamburg, 20148 Hamburg (DE); Universitätsklinikum Hamburg-Eppendorf, 20246 Hamburg (DE)
(72) Erfinder: Faryammanesh, Rassa, 22335 Hamburg (DE); Hahn, Ulrich, 22391 Hamburg (DE); Schumacher, Udo, 22085 Hamburg (DE); Wicklein, Daniel, 22159 Hamburg (DE)
(74) Vertreter: Stüven, Ralf

(56) Entgegenhaltungen:
- WO-A2-2004/082458
- WO-A2-2005/014857
- WO-A2-2009/140383
- US-A1- 2012 039 810
- Rassa Faryammanesh: "Selektion von DNA-Aptameren", Innovative Medizin- und Biotechnologie aus Hamburg, 9. Hamburger Studententagung, 2. Mai 2012 (2012-05-02), XP55173222, Gefunden im Internet: URL:http://www.baytobio.de/newsticker/2012 /05/Tagungsband_Studententagung.pdf [gefunden am 2015-03-03]
- CINDY MEYER ET AL: "Cell-Specific Aptamers as Emerging Therapeutics", JOURNAL OF NUCLEIC ACIDS, Bd. 108, Nr. 10, 28. August 2011 (2011-08-28), Seiten 3900-18, XP55173341, DOI: 10.1021/ja043285e
- KRISTINA W. THIEL ET AL: "Therapeutic Applications of DNA and RNA Aptamers", OLIGONUCLEOTIDES, Bd. 19, Nr. 3, 5. August 2009 (2009-08-05) , Seiten 209-222, XP055078801, ISSN: 1545-4576, DOI: 10.1089/oli.2009.0199
- AMAN P. MANN ET AL: "Identification of Thioaptamer Ligand against E-Selectin: Potential Application for Inflamed Vasculature Targeting", PLOS ONE, Bd. 5, Nr. 9, 30. September 2010 (2010-09-30), Seite e13050, XP55173310, DOI: 10.1371/journal.pone.0013050
- ROBERT D. JENISON ET AL: "Oligonucleotide Inhibitors of P-Selectin-Dependent Neutrophil-Platelet Adhesion", ANTISENSE AND NUCLEIC ACID DRUG DEVELOPMENT, Bd. 8, Nr. 4, August 1998 (1998-08), Seiten 265-279, XP55173327, ISSN: 1087-2906, DOI: 10.1089/oli.1.1998.8.265
- W. ZHAO ET AL: "Mimicking the inflammatory cell adhesion cascade by nucleic acid aptamer programmed cell-cell interactions", THE FASEB JOURNAL, Bd. 25, Nr. 9, 7. Juni 2011 (2011-06-07), Seiten 3045-3056, XP55173324, ISSN: 0892-6638, DOI: 10.1096/fj.10-178384
- D. R. GUTSAEVA ET AL: "Inhibition of cell adhesion by anti-P-selectin aptamer: a new potential therapeutic agent for sickle cell disease", BLOOD, Bd. 117, Nr. 2, 6. Oktober 2010 (2010-10-06), Seiten 727-735, XP55173307, ISSN: 0006-4971, DOI: 10.1182/blood-2010-05-285718
- RINGQUIST S ET AL: "ANTI-L-SELECTIN OLIGONUCLEOTIDE LIGANDS RECOGNIZE CD62L-POSITIVE LEUKOCYTES: BINDING AFFINITY AND SPECIFICITY OF UNIVALENT AND BIVALENT LIGANDS", CYTOMETRY, ALAN LISS, NEW YORK, US, Bd. 33, 12. Dezember 1998 (1998-12-12), Seiten 394-405, XP002927384, ISSN: 0196-4763, DOI: 10.1002/(SICI)1097-0320(19981201)33:4<394: :AID-CYTO2>3.0.CO;2-0
- RASSA FARYAMMANESH ET AL: "SDA, a DNA Aptamer Inhibiting E- and P-Selectin Mediated Adhesion of Cancer and Leukemia Cells, the First and Pivotal Step in Transendothelial Migration during Metastasis Formation", PLOS ONE, Bd. 9, Nr. 4, 3. April 2014 (2014-04-03), Seite e93173, XP55173336, DOI: 10.1371/journal.pone.0093173
- Rassa Faryammanesh: "Selektion und Charakterisierung Selektin-spezifischer DNA- Aptamere", Ph. D. thesis, 2014, XP55173330, Hamburg, DE Gefunden im Internet: URL:http://ediss.sub.uni-hamburg.de/vollte xte/2014/6805/pdf/Dissertation.pdf [gefunden am 2015-03-03]

### SEQUENCE LISTING

<110> UniversitÃ¤t Hamburg
   UniversitÃ¤tsklinikum Hamburg-Eppendorf (UKE)
<120> DNA-Aptamere, die E- und P-Selektine spezifisch binden
<130> PAT 1464 EP
<150> DE102013112915.2
   <151> 2013-11-22
<160> 5
<170> BiSSAP 1.3
<210> 1
   <211> 91
   <212> DNA
   <213> Artificial Sequence
<220>
   <223> DNA aptamer SDA1
<400> 1
   gcctgttgtg agcctcctaa ccgcgaattc gtgtcactaa acaccgatag tttcgattgt 60
   ctttggttca tcatgcttat tcttgtctcc c 91
<210> 2
   <211> 91
   <212> DNA
   <213> Artificial Sequence
<220>
   <223> DNA aptamer SDA2
<400> 2
   gcctgttgtg agcctcctaa cgatttggat ttggggtgga gggtatggtt tgtgctggcg 60
   ttctcatttc ccatgcttat tcttgtctcc c 91
<210> 3
   <211> 20
   <212> DNA
   <213> Artificial Sequence
<220>
   <223> Reverse primer
<400> 3
   gggagacaag aataagcatg 20
<210> 4
   <211> 21
   <212> DNA
   <213> Artificial Sequence
<220>
   <223> Forward primer
<400> 4
   gcctgttgtg agcctcctaa c 21
<210> 5
   <211> 91
   <212> DNA
   <213> Artificial Sequence
<220>
   <223> SDA2 scramble variant
<400> 5
   attctaaggc gttaccccaa tggatttccg tcggaattgc tatagccctt gaacgctaca 60
   tgcacgatac caagttatgt atggaccggg t 91

## Beschreibung

Die Erfindung betrifft Aptamere, die E- und P-Selektine spezifisch binden.

Selektine sind membranständige Glykoproteine, die an spezifische Oligosaccharide in Glykoproteinen und Glykolipiden, die auf Zelloberflächen benachbarter Zellen exprimiert werden, binden und damit beispielsweise eine transiente Adhäsion von weißen Blutkörperchen an Endothelzellen vermitteln. Diese Adhäsion ist wichtig für den Austritt (Extravasation) von Leukozyten aus der Blutbahn ins Interstitium und damit für deren Rekrutierung zu Entzündungsherden sowie das Lymphozyten-"Homing".

Selektine spielen aber auch eine entscheidende Rolle bei der Metastasierung von malignen Tumoren. Dabei lösen sich Tumorzellen aus dem Verband des Primärtumors heraus und infiltrieren die umgebende Extrazellulärmatrix und die Tumorblutgefäße. Mit dem Blutstrom gelangen diese Tumorzellen dann zu einem anderen Organ, dem Sitz der späteren Metastase. Dort haften sie, vermittelt durch Selektine, an das Endothel an, durchwandern es und proliferieren, um schließlich eine klinisch detektierbare Metastase zu bilden. Nach Erreichen einer gewissen Metastasengröße kann sich der Vorgang wiederholen. Diese hämatogene Metastasierung ist bisher klinisch nicht beherrschbar. Entsprechendes gilt auch für die intraperitoneale Metastasierung. Auch sie wird durch Selektine vermittelt und ist klinisch schwer bzw. gar nicht zu behandeln.

Selektine werden nach den sie exprimierenden Zelltypen in L-, E- und P-Selektine gruppiert. L-Selektine werden in Lymphozyten, Granulozyten und Monozyten, E-Selektine in Endothelzellen und P-Selektine in Blutplättchen und Endothelzellen exprimiert.

Aptamere sind kurze synthetische einzelsträngige DNA- oder RNA-Oligonukleotide mit der Fähigkeit zur hochaffinen spezifischen Bindung an Zielmoleküle, beispielsweise Proteine. Ihre antikörperähnlichen Bindungseigenschaften machen Aptamere für den Einsatz als potenzielle Arzneimittel attraktiv (s. z.B. Osborne et al. (1997), Curr. Opin. Chem. Biol. 1, 5-9, Meyer, C., et al. 2011, Journal of Nucleic Acids, doi:10.4061/2011/904750; Keefe, A.D., et al. 2010, Nat Rev Drug Discov. 9: 537-50, doi:10.1038/nrd3141; Kanwar et al. 2011, Crit Rev Biochem Mol Biol. 46: 459-477, doi:10.3109/10409238.2011.614592; Thiel, K.W., Giangrande, P.H., 2009, Oligonucleotides 19, 209-222. doi: 10.1089/oli.2009.0199). Gegenüber Antikörpern weisen Aptamere bei hoher Spezifität und Affinität sowie chemischer Stabilität eine niedrige Immunogenität auf. Aptamere können zum Beispiel mit Hilfe eines Verfahrens selektiert werden, das als SELEX (systematic evolution of ligands by exponential enrichment) bezeichnet wird (Ellington und Szostak (1990), Nature 346, 818-822; Gopinath (2007), Anal. Bioanal. Chem. 387, 171-182; WO 91/19813).

Aptamere, die E- oder P-Selektine binden, sind im Stand der Technik grundsätzlich bekannt (s. Gutsaeva, D.R., 2011, Blood 117: 727-735, doi:10.1182/blood-2010-05-285718; Mann et al. 2010, PLoS ONE 5(9): e13050. doi:10.1371/journal.pone.0013050; US 2012/0039810 A1; Meyer, C., et al. 2011, Journal of Nucleic Acids, doi:10.4061/2011/904750; Faryammanesh, R., 2012, Selektion von DNA-Aptameren, In: Innovative Medizin- und Biotechnologie aus Hamburg, 9. Hamburger Studententagung 02.05.2012, S. 50-51; Zhao, W., 2011, FASEB J. 25, 3045-56, doi: 10.1096/fj.10-178384). In der WO 2013/096926 A1 sind Aptamere als mögliche E-Selektin-Antagonisten angegeben. Geeignete Aptamersequenzen sind jedoch nicht offenbart. In der WO 2009/090554 A2 sind Aptamere beschrieben, die zur Verhinderung der metastatischen Krebszellmigration erzeugt wurden. Die WO 2009/140383 A2 beschreibt RNAbasierte Aptamere, die humanes P-Selektin spezifisch binden. P-Selektin-spezifische RNA-Aptamere sind auch in Jenison R.D., 1998, Antisense Nucleic Acid Drug Dev. 8, 265-279, beschrieben.

Nach wie vor besteht ein Bedarf an Mitteln, mit deren Hilfe krankhafte Zustände behandelt werden können, bei denen die Adhäsion von Zellen an Endothelzellen eine Rolle spielt, beispielsweise bei Entzündungsprozessen, regenerativen Prozessen oder der Metastasenbildung. Aufgabe der vorliegenden Erfindung ist es, solche Mittel bereitzustellen.

Gelöst wird die Aufgabe durch die Gegenstände des Anspruchs 1 und der weiteren nebengeordneten Ansprüche. Vorteilhafte Ausgestaltungen der Erfindung sind in den Unteransprüchen angegeben.

In einem ersten Aspekt stellt die Erfindung ein DNA-Aptamer bereit, das menschliches E-Selektin und/oder P-Selektin spezifisch bindet und
a) eine Sequenz mit den Nukleotiden 22-71 gemäß SEQ ID NO: 1 oder SEQ ID NO: 2, oder
b) eine Sequenz mit mindestens 75%, 80%, 85%, 90%, 95%, 96%, 97%, 98% oder 99% Identität zu einer Sequenz mit den Nukleotiden 22-71 gemäß SEQ ID NO: 1 oder SEQ ID NO: 2, oder
c) eine Sequenz gemäß a) oder b) mit mindestens einem modifizierten Nukleotid umfasst, wobei das mindestens eine modifizierte Nukleotid ein 2'-Desoxy, 2'-Halogenid-, 2'-Amino-, 2'-O-Methyl- oder 2'-Methoxyethyl-Nukleotid ist.

Das erfindungsgemäße DNA-Aptamer ist affin und spezifisch für E- und P-Selektin, und dabei voraussichtlich nicht oder wenig immunogen. Es stellt ein viel versprechendes Mittel bereit, um Erkrankungen, an denen E- und/oder P-Selektine beteiligt sind, zu behandeln oder zu verhindern. Beispielsweise ist es vorteilhaft einsetzbar zur Verhinderung oder Minimierung einer Metastasenbildung bei Krebs, indem es die Extravasation von Tumorzellen unterbindet oder zumindest erschwert. Weitere Beispiele sind ein Einsatz zur Behandlung von Entzündungsvorgängen und/oder zur Förderung regenerativer Prozesse, z.B. bei Rheuma, und/oder ein Einsatz zur Beeinflussung der Mobilisierung und/oder des "Homing" von Stammzellen, insbesondere hämatopoetischer Stammzellen, beispielsweise im Fall von Stammzelltransplantationen. Das erfindungsgemäße DNA-Aptamer ist darüber hinaus einfach und kostengünstig herzustellen, gut haltbar und lagerungsfähig.

Die Sequenzen gemäß SEQ ID NO: 1 und 2 umfassen jeweils 91 Nukleotide, wobei jeweils identische Teilsequenzen von 21 Nukleotiden am 5'-Ende (nt 1-21) und von 20 Nukleotiden am 3'-Ende (72-91) einen randomisierten Sequenzabschnitt von 50 nt flankieren.

Das erfindungsgemäße DNA-Aptamer kann auch verwendet werden, um E-Selektin oder P-Selektin oder Zellen, die E-Selektin oder P-Selektin präsentieren, darzustellen, nachzuweisen und/oder zu isolieren. Hierzu kann das DNA-Aptamer beispielsweise mittels bekannter Techniken immobilisiert und in dieser immobilisierten Form als Bindemittel, beispielsweise für eine Säulenchromatographie, verwendet werden. Beispielsweise kann das erfindungsgemäße Aptamer mittels eines dem Fachmann bekannten Kopplungssystems, z.B. des Avidin-Biotion-oder Biotin-Streptavidin-Systems, multimerisiert und gegebenenfalls an Nanopartikel, z.B. so genannte Quantenpunkte ("Quantum Dots", QD), gebunden werden. Mittels geeigneter Fluoreszenzmarkierung oder anderer dem Fachmann bekannter Techniken kann das erfindungsgemäße DNA-Aptamer auch zu Bildgebungszwecken eingesetzt werden.

Unter einem "DNA-Aptamer" wird hier eine isolierte Einzelstrang-DNA (ssDNA) verstanden, die ein Zielmolekül, z.B. ein Protein, spezifisch bindet. Insbesondere werden unter dem Begriff "DNA-Aptamer" ssDNA-Oligonukleotide (Einzelstrang-Oligonukleotide) mit höchstens 150, vorzugsweise höchstens 130, höchstens 110, höchstens 100, höchstens 90, höchstens 80, höchstens 70, höchstens 60, höchstens 50, höchstens 40, höchstens 30, höchstens 20, höchstens 15 oder höchstens 10 Nukleotiden verstanden. Insbesondere werden unter dem Begriff ssDNA-Oligonukleotide mit 15-100 Nukleotiden verstanden.

Unter einem "Nukleotid" werden hier insbesondere die Grundbausteine von Nukleinsäuren, d.h. organische Moleküle verstanden, die aus einem Zuckerrest, in der Regel einer Pentose, z.B. Desoxyribose oder Ribose, einer organischen Base (Nukleobase) und Phosphorsäure bestehen. Die Phosphorsäure ist mit dem Zucker regelmäßig über eine Esterbindung, der Zucker mit der Nukleobase über eine N-glykosidische Bindung verbunden. In Desoxyribonukleinsäure (DNA) kommen regelmäßig die Nukleobasen Adenin (A), Cytosin (C), Guanin (G) und Thymin (T) vor, während in Ribonukleinsäure (RNA) anstelle des Thymins die Base Uracil (U) vertreten ist. Die Phosphorsäure liegt in RNA und DNA in der Regel als Monophosphat vor. Die Verknüpfung von Nukleotiden untereinander erfolgt meistens über eine Phosphordiesterbindung zwischen dem 5'-C-Atom einer Pentose und dem 3'-C-Atom einer benachbarten Pentose. Phosphorthioat- oder Phosphorselenoat-Nukleotide sind aber auch von dem Begriff "Nukleotid" umfasst, d.h. Nukleotide, bei denen die Phosphorsäure durch z.B. Phosphorthioat, Phosphordithioat oder Phosphorselenoat ersetzt ist.

Unter einem "modifizierten Nukleotid" wird hier ein Nukleotid verstanden, das chemisch gegenüber dem ursprünglichen Nukleotid modifiziert ist. Beispiele für modifizierte Nukleotide sind 2'-Desoxy-, 2'-Halogenid- (z.B. 2'-F-), 2'-Amino-, 2'-O-Methyl-Nukleotide oder 2'-Methoxyethyl-Nukleotide, d.h. Nukleotide, die am 2'-C-Atom der Zuckerkomponente anstelle einer OH-Gruppe Wasserstoff, ein Halogenid (z.B. Fluor), eine Amino-, O-Methyl- oder Methoxyethyl-Gruppe aufweisen. Der Begriff umfasst aber auch Nukleotide, die an der Basenkomponente modifiziert sind.

Das Merkmal, wonach das DNA-Aptamer "eine Sequenz gemäß a) oder b) mit mindestens einem modifizierten Nukleotid" umfasst, bedeutet, dass das DNA-Aptamer eine Sequenz umfasst, die ursprünglich einer der in den Merkmalen a) oder b) genannten Sequenzen entspricht, wobei jedoch mindestens eines der Nukleotide in modifizierter Form vorliegt, z.B. in Form eines 2'-F-Nukleotids. Die Formulierung, dass "mindestens ein Nukleotid" modifiziert wurde bzw. in modifizierter Form vorliegt, schließt Modifikationen an einem oder mehreren Nukleotiden ein, wobei es sich nicht jeweils um dieselbe Modifikation handeln muss, sondern unterschiedliche Modifikationen vorliegen können, beispielsweise eine 2'-F-Modifikation an einem Nukleotid, eine 2'-Desoxy-Modifikation an einem zweiten Nukleotid und eine 2'-O-Methyl-Modifikation an einem dritten Nukleotid.

Die Angabe einer Identität von Nukleotid- oder Aminosäuresequenzen in Zusammenhang mit einer Prozentangabe, z.B. "x% Identität", bezieht sich auf einen Vergleich zweier Sequenzen, wobei jeweils eine Position in der einen Sequenz mit der entsprechenden Position in der anderen Sequenz verglichen wird, und bedeutet eine Identität der Nukleotide oder Aminosäuren der beiden verglichenen Sequenzen in x% der verglichenen Positionen. Dabei kann es gegebenenfalls erforderlich sein, Sequenzlücken zu berücksichtigen, um eine möglichst gute Alinierung der Vergleichssequenzen herzustellen. Identität heißt demnach, dass beim Vergleich zweier Sequenzen an äquivalenten Stellen jeweils dasselbe Nukleotid bzw. dieselbe Aminosäure steht. Der Grad der Ähnlichkeit oder Identität zweier Sequenzen kann beispielsweise mit Hilfe des Computerprogramms BLAST (S.F. Altschul et al. (1990), Basic Local Alignment search tool, J.Mol. Biol. 215: 403-410; s. z.B. http://www.ncbi.nlm.nih.gov/BLAST/) unter Verwendung von Standardparametern ermittelt werden, wobei dem Fachmann geläufig ist, welches Programm für die jeweilige Sequenz geeignet ist (z.B. BLASTn für Nukleotid-, BLASTp für Aminosäuresequenzen).

Die Formulierung "Sequenz mit mindestens x% Identität zu einer Sequenz mit den Nukleotiden y-z" bedeutet hier, dass beim Sequenzvergleich eine Sequenz eingesetzt wird, die aus den Nukleotiden von Position y bis Position z der in Bezug genommenen Sequenz besteht, z.B. aus den Nukleotiden der Positionen 22-71.

Der Begriff "E-Selektin" hat hier die im Stand der Technik bekannte Bedeutung und bezieht sich auf ein Zelladhäsionsmolekül, das auch als SELE, CD62E, ELAM, ELAM1, ESEL oder LECAM2 bezeichnet wird (GenBank-Zugriffsnummer für den humanen E-Selektin-Präkursor: NM_000450 (mRNA) und NP_000441 (Protein)).

Der Begriff "P-Selektin" hat die im Stand der Technik bekannte Bedeutung und bezieht sich auf ein Zelladhäsionsmolekül, das auch als, SELP, CD62, CD62P, FLJ45155, GMP140, GRMP, PADGEM oder PSEL bezeichnet wird (GenBank-Zugriffsnummer für den menschlichen P-Selektin-Präkursor: NM_003005 (mRNA) and NP_002996 (Protein)).

E-Selektin und P-Selektin binden an sialylierte, insbesondere Neuraminsäure-substituierte, Kohlenhydrate vom Lewis-X- und/oder Lewis-A-Typ auf Gykoproteinen als Liganden, beispielsweise an Glykoproteine mit der sialylierten Lewis^{x}-Gruppe SLe^{x} (NeuAcα2-3Galβ1-4(Fucα1-3)GlcNAc oder der sialylierten Lewis^{A}-Gruppe SLe^{a} (NeuAcα2-3Galβ1-3(Fucα1-4)GlcNAc (NeuAc = N-Acetylneuraminsäure, Gal = Galactose, Fuc = Fucose, GlcNAc = N-Acetylglucosamin).

Unter einem humanes E-Selektin oder P-Selektin spezifisch bindenden DNA-Aptamer oder einem Fragment davon wird hier ein DNA-Aptamer oder DNA-Aptamer-Fragment verstanden, das mit humanem E-Selektin oder P-Selektin eine Dissoziationskonstante (K_{d}) von maximal 7•10⁻⁶ M (mol/1), bevorzugt maximal 5•10⁻⁶ M, bevorzugt maximal 3•10⁻⁶ M, bevorzugt maximal 2•10⁻⁶ M, bevorzugt maximal 10⁻⁶ M, maximal 8•10⁻⁷ M, maximal 5•10⁻⁷ M, maximal 3•10⁻⁷ M, maximal 10⁻⁷ M, maximal 8•10⁻⁸ M, maximal 5•10⁻⁸ M oder maximal 3•10⁻⁸ M aufweist. Insbesondere wird unter einem humanes E-Selektin oder P-Selektin spezifisch bindenden DNA-Aptamer oder DNA-Aptamer-Fragment ein DNA-Aptamer oder DNA-Aptamer-Fragment verstanden, das eine Dissoziationskonstante von maximal 1000 nM (nmol/1), vorzugsweise maximal 500 nM, weiter bevorzugt maximal 250 nM, maximal 200 nM, maximal 150 nM, maximal 120 nM, maximal 110 nM und besonders bevorzugt maximal 100 nM aufweist. K_{d}-Werte können beispielsweise mit Hilfe von radioaktiven Filterbindungsstudien unter Verwendung eines "One site - specific-binding"-Modells und unter Zuhilfenahme des Programms GraphPad® Prism (GraphPad Software Inc., La Jolla, CA 92037 USA) ermittelt werden (s. z. B. Meyer, C., Eydeler, K., Magbanua, E., Zivkovic, T., Piganeau, N., Lorenzen, I., Grötzinger, J., Mayer, G., Rose-John, S. & Hahn, U. (2012) Interleukin-6 receptor specific RNA aptamers for cargo delivery into target cells. RNA Biology 9, 67-80, doi: 10.4161/rna.9.1.18062). Die obigen Angaben zu K_{d}-Werten beziehen sich auf Mittelwerte. Unter einem Fragment eines humanes E-Selektin oder P-Selektin spezifisch bindenden DNA-Aptamers wird hier nur ein ebenfalls humanes E-Selektin oder P-Selektin spezifisch bindendes Fragment eines DNA-Aptamers verstanden. Vorzugsweise stammt das Fragment ausschließlich aus dem randomisierten Teil des DNA-Aptamers.

In einer bevorzugten Ausführungsform hat oder umfasst das erfindungsgemäße DNA-Aptamer eine der Sequenzen gemäß SEQ ID NO: 1 oder SEQ ID NO: 2. Die Formulierung, dass das DNA-Aptamer eine Sequenz "hat", bedeutet, dass das DNA-Aptamer aus der Sequenz besteht, d.h. am 3'- und 5'-Ende keine weiteren Nukleotide vorhanden sind.

Eine oder mehrere, gegebenenfalls auch alle Basen der Nukleotide des DNA-Aptamers oder des DNA-Aptamer-Fragments können modifiziert sein. Dies kann vorteilhaft sein, um beispielsweise die Empfindlichkeit gegenüber Nukleasen in vivo zu verringern, die Aufnahme in die Zelle zu verbessern oder eine schnelle renale Resorption zu verhindern. Die Vornahme entsprechender Modifikationen liegt im Bereich des Fachkönnens des Durchschnittsfachmanns.

Das erfindungsgemäße DNA-Aptamer kann auch mit anderen Verbindungen, z.B. Cholesterol oder Polyethylenglykol (PEG), gekoppelt oder auch multimerisiert werden, um beispielsweise die Bioverfügbarkeit oder die Affinität zu erhöhen, den Abbau oder die Ausscheidung zu vermindern. Zum Schutz vor dem Angriff von Exonukleasen kann beispielsweise am 3'-Ende eine 3'-3'-dT-Kappe (dT = Desoxythymidin) vorgesehen sein.

In einer bevorzugten Ausführungsform ist das DNA-Aptamer PEGyliert. "PEGyliert" bedeutet, dass das DNA-Aptamer mit einem Polyethylenglycol(PEG)-Polymer chemisch verbunden (konjugiert) ist, beispielsweise am 5'-Ende (s. z.B. US 7803931 B1). Es kann sich beispielsweise um ein lineares PEG-Polymer von 10 kDa, 20 kDa, 30 kDa oder 40 kDa handeln. Auch verzweigte PEG-Polymere können eingesetzt werden. PEGylierungsverfahren und geeignete PEG-Polymere sind dem Fachmann bekannt (s. z.B. WO 2005084412 A2, US 7803931 B1).

In einem weiteren Aspekt stellt die Erfindung auch eine Nukleinsäure bereit, umfassend oder bestehend aus einem Oligomer eines erfindungsgemäßen DNA-Aptamers. Das erfindungsgemäße DNA-Aptamer kann somit auch oligomerisiert, d.h. als Oligomer des erfindungsgemäßen DNA-Aptamers, vorliegen. Unter einem Oligomer eines DNA-Aptamers (DNA-Aptamer-Oligomer) wird hier eine Nukleinsäure verstanden, die aus mindestens zwei wiederholt auftretenden DNA-Aptamer-Einheiten (Monomeren) zusammengesetzt ist. Bei dem Monomer kann es sich um das vollständige Aptamer einschließlich eines oder beider invariabler Randbereiche oder um den vollständigen randomisierten Teil des Aptamers oder um E- oder P-Selektin-bindende Fragmente des Aptamers handeln. Die Nukleinsäure kann auch ein Mischoligomer sein, d.h. aus zwei oder mehreren nicht identischen Wiederholungseinheiten zusammengesetzt sein, beispielsweise mindestens einem in SEQ ID NO:1 angegebenen DNA-Aptamer und mindestens einem in SEQ ID NO:2 angegebenen DNA-Aptamer. Die Wiederholungseinheiten müssen nicht unmittelbar aufeinander folgen, sondern können auch durch ein oder mehrere Nukleotide voneinander getrennt sein. Vorzugsweise ist das DNA-Aptamer-Oligomer aus zwei, drei, vier, fünf, sechs, sieben, acht, neun oder zehn Wiederholungseinheiten zusammengesetzt. Ein solches DNA-Aptamer-Oligomer kann vorteilhaft beispielsweise auf Humanserumalbumin immobilisiert sein.

Das erfindungsgemäße DNA-Aptamer oder DNA-Aptamer-Oligomer kann vorteilhaft als Arzneimittel, vorzugsweise gegen Entzündungen und/oder Krebs und/oder zur Förderung regenerativer Prozesse wie der Geweberegeneration, verwendet werden. Hierzu kann das DNA-Aptamer oder DNA-Aptamer-Oligomer in für den Fachmann bekannter Weise in einer Zusammensetzung enthalten sein, die in einer geeigneten Verabreichungsform formuliert wird. Das DNA-Aptamer oder DNA-Aptamer-Oligomer wird dabei in einer pharmazeutisch wirksamen Menge verwendet, d.h. einer Menge, die eine nachweisbare Wirkung auf den behandelten Zustand ausübt.

In einem weiteren Aspekt stellt die vorliegende Erfindung ein Arzneimittel bereit, das ein erfindungsgemäßes DNA-Aptamer oder DNA-Aptamer-Oligomer umfasst. Das Aptamer oder Aptamer-Oligomer ist dabei in einer pharmazeutisch wirksamen Menge in dem Arzneimittel enthalten. Das Arzneimittel umfasst darüber hinaus bevorzugt geeignetes Trägermaterial, Exzipientien und dergleichen. Gegebenenfalls kann das Arzneimittel auch einen oder mehrere weitere Wirkstoffe enthalten. Die Wirkstoffe können dabei auch an das DNA-Aptamer oder Aptamer-Oligomer gekoppelt, d.h. kovalent oder nicht-kovalent gebunden sein. Geeignete Formulierungen und Darreichungsformen sind dem Fachmann bekannt oder können auf routinemäßige Weise gemäß dem Stand der Technik hergestellt werden. Die erfindungsgemäßen Aptamere oder Aptamer-Oligomere können beispielsweise auch an Nanopartikel gebunden werden, die mit anderen Wirkstoffen beladen sind, wodurch eine gezielte Zufuhr der Wirkstoffe ermöglicht wird.

Die Erfindung wird im Folgenden anhand von Ausführungsbeispielen und den beigefügten Figuren zu Veranschaulichungszwecken näher erläutert.

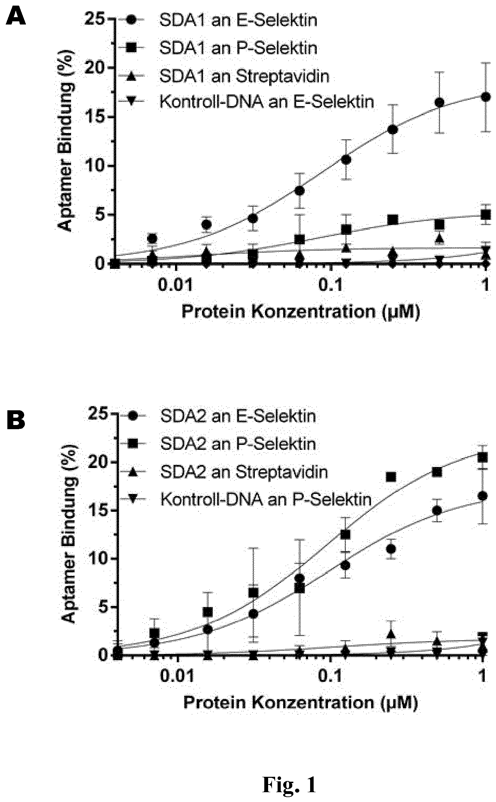

Figur 1 Affinität der erfindungsgemäßen DNA-Aptamere gegenüber E- und P-Selektin, ermittelt durch Filter-Retentions-Assays (FRA). DNA wurde radiomarkiert, mit ansteigenden Mengen Protein (E- und P-Selektin, Streptavidin als Kontrolle) inkubiert und durch eine Nitrocellulosemembran filtriert. Gebundenes Aptamer wurde mittels Autoradiographie detektiert und quantifiziert. A. SDA1 inkubiert mit humanem E-Selektin (●) und humanem P-Selektin (■) oder Streptavidin (▲) als Kontrolle; Kontroll-DNA (▼). B. SDA2 inkubiert mit humanem E-Selektin (●) und humanem P-Selektin (■) oder Streptavidin (▲) als Kontrolle; Kontroll-DNA statt DNA-Aptamer (▼).

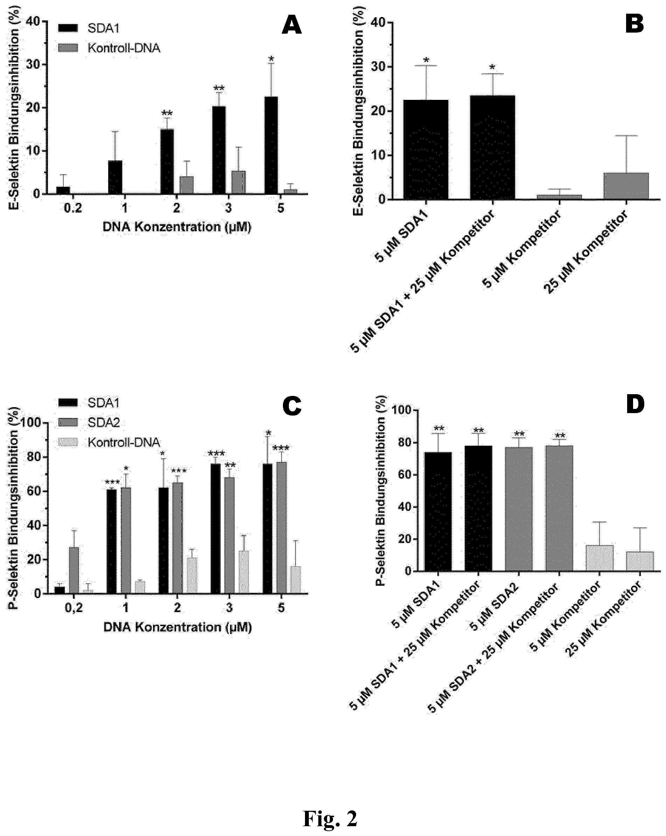

Figur 2 Aptamer-vermittelte Hemmung der statischen Interaktion zwischen Selektinen und HT29-Zellen. Fig. 2A, B: Hemmung der Interaktion von rhE-Selektin mit HT29-Zellen durch SDA1 sowie Einfluss eines Kompetitors auf die Interaktion (B). Fig. 2C, D: Hemmung der Interaktion von rhP-Selektin mit HT29-Zellen durch SDA1 und SDA2 sowie Einfluss eines Kompetitors auf die Interaktion (D). Die Aptamere wurden vor Inkubation mit HT29-Zellen mit PE-markiertem rekombinantem humanem (rh)-E- oder -P-Selektin präinkubiert. Die relative Bindung von HT29-Zellen wurde mittels Durchflusszytometrie in drei unabhängigen Experimenten bestimmt. P-Werte wurden ermittelt durch Vergleich gegebener Werte mit Aptamer-Hemmung bei 0.2 µM (A und C) oder 5 µM Kompetitor-Konzentration (B und D); * P < 0.05, ** P < 0.01, *** P < 0.001.

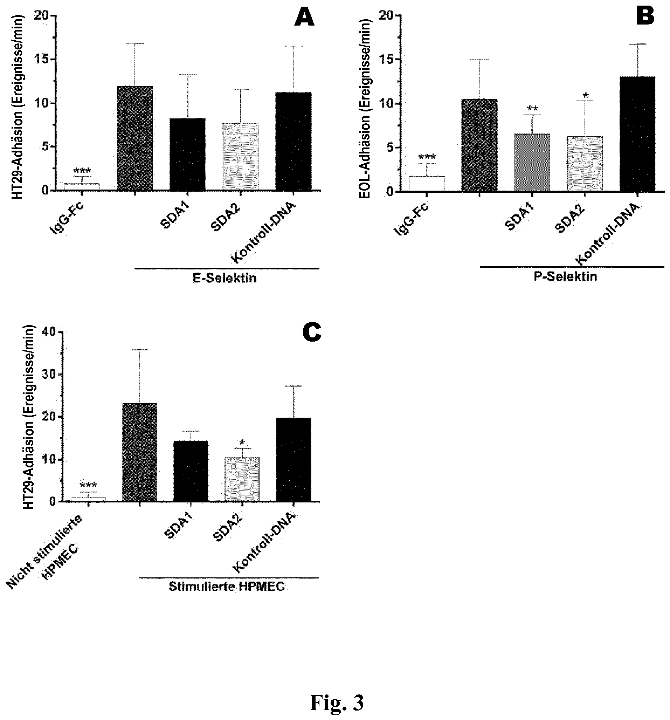

Figur 3. Aptamer-Hemmung der dynamischen Zelladhäsion. Immobilisierte rhE- (A) oder rhP-(B) Selektin/IgG-Fc-Chimären (je 0,2 µM) sowie stimulierte MPMECs (C) wurden mit 5 µM des jeweiligen Aptamers inkubiert. Selektin-Ligand-präsentierende Tumorzellen wurden über immobilisierte Proteine oder E-Selektin präsentierende HPMECs perfundiert (Flussrate 8 mL/h) und die zurückgehaltenen Zellen wurden gezählt. Beide Aptamere reduzierten die Adhäsion zu entsprechenden Zellen (n = 6 von insgesamt 2 verschiedenen Experimenten, P-Werte angewendet auf die Selektine. Die IgG-F_{C}-Kontrolle zeigte keine Adhäsionswirkung. Alle P-Werte wurden mit unbehandelten Selektinen als Standard ermittelt (* P < 0.05, ** P < 0.01, *** P < 0.001).

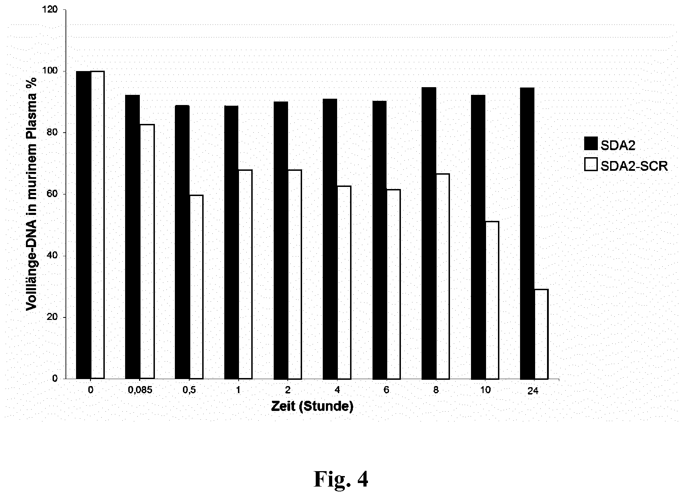

Figur 4. Stabilität in Plasma. Darstellung der mittels denaturierender Polyacrylamid-Gelelektrophorese ermittelten Stabilität von SDA2 und einer "Scramble"-Variante davon (SDA2-SCR) in murinem Plasma. Die radioaktiv markierten Nukleinsäuren wurden für definierte Zeiträume bei 37°C in murinem Plasma inkubiert, in flüssigem Stickstoff zwischengelagert und anschließend mittels PAGE analysiert. Schwarz: SDA2, weiß: SDA2-SCR.

### Beispiele

Aptamere mit hoher Affinität für E- und P-Selektin wurden selektiert und mittels Filter-Retentions-Assays (FRA) auf ihre Bindung gegenüber den Zielmolekülen untersucht. Zur Untersuchung der Fähigkeit der Aptamere, die Wechselwirkung von Krebs- und Leukämie-Zellen mit Selektinen zu blockieren, wurden laminare Strömungsassays unter physiologischer Scherbeanspruchung durchgeführt. Es wurden menschliche HT29-Dickdarmkrebszellen, menschliche EOL-1-Zellen (chronische eosinophile Leukämie, CEL) und humane primäre pulmonale mikrovaskuläre Endothelzellen (HPMECs) eingesetzt.

### 1. In-vitro-Selektion von DNA-Aptameren

Zur Selektion von DNA-Aptameren wurde ein als SELEX (Systematische Evolution von Liganden durch exponentielle Anreicherung) bezeichneter In-vitro-Selektionsprozess eingesetzt (Ellington und Szostak (1990), Nature 346, 818-822; Gopinath (2007), Anal. Bioanal. Chem. 387, 171-182; WO 91/19813; Tuerk C, Gold L. (1990), Systematic Evolution of Ligands by Exponential Enrichment: RNA Ligands to Bacteriophage-T4 DNA-Polymerase, Science 249 (4968):505-510, DOI 10.1126/science.2200121). Der Selektionsprozess erfolgt in iterativen Zyklen der folgenden Schritte: (1) Inkubation einer Nukleinsäure-Bibliothek mit dem Zielmolekül, (2) Trennung bindender von nicht-bindenden Nukleinsäuren und (3) Vervielfältigung bindender Spezies, d.h. eine Anreicherung von bindenden Nukleinsäuren.

### 1.1 Biotinylierung von E-Selektin und Immobilisierung auf Streptavidin-beschichteten Dynabeads® ("Target"-Kügelchen)

Für die Biotinylierung wurden 50 µg rekombinante humane E-Selektin/IgG-Fc-Chimären (rhE-Selektin, R&D Systems) mit dreifachem molaren Überschuss von Sulfo-NHS-LC-Biotin (Thermo Scientific) nach Herstellerangaben inkubiert. Schließlich wurde das biotinylierte rhE-Selektin auf 5 mg Streptavidin-beschichteten Magnetkügelchen (Dynabeads®, Life Technologies) immobilisiert und in Selektionspuffer (3 mM MgCl₂ in phosphatgepufferter Salzlösung (PBS), pH 7,5) mit 1 mg Rinderserumalbumin (BSA) pro ml suspendiert (Mayer G, Hover T. In vitro selection of ssDNA aptamers using biotinylated target proteins. Methods Mol Biol. 2009;535:19-32. Vorveröffentlicht am 2009/04/21 als DOI 10.1007/978-1-59745-557-2_2).

### 1.2 Biotin-Immobilisierung auf Streptavidin-beschichteten Dynabeads® (PräselektionsKügelchen)

Streptavidin-beschichtete Magnetkügelchen (5 mg) wurden fünfmal mit 500 µl Waschpuffer (1 mg BSA / ml PBS) gewaschen und in 500 µl 0,9 mM PBS-Biotin-Lösung resuspendiert. Nach 30 Minuten Inkubation bei Raumtemperatur wurden die Biotin-Streptavidin-beschichteten Magnetkügelchen fünfmal mit 500 µl Waschpuffer gewaschen und in 1,5 ml PBS mit 1,25 mg BSA/ml.

### 1.3 In-vitro-Selektion von Selektin-bindenden DNA-Aptameren

Für die In-vitro-Selektion von E-Selektin spezifischen DNA-Aptameren wurde das SELEX-Verfahren verwendet (s.o.), einschließlich eines Präselektionsschrittes zur Entfernung Streptavidin-bindendender DNA. Die ssDNA-Ausgangsbibliothek wies dabei folgende Grundstruktur auf: 5'-GCCTGTTGTGAGCCTCCTAAC-N50-CATGCTTATTCTTGTCTCCC-3'

Die Sequenzen der ssDNA-Bibliothek waren charakterisiert durch einen randomisierten Bereich von 50 Nukleotiden, welcher sowohl am 3'- als auch am 5'-Ende von konstanten Regionen mit 21 bzw. 20 Nukleotiden Länge flankiert war. Die konstanten Regionen dienten zur Anlagerung der PCR-Primer.

500 pmol dieser Ausgangsbibliothek wurden für 30 Minuten bei Raumtemperatur mit 30 nmol Präselektionskügelchen inkubiert, und Streptavidin-bindende DNA wurde über magnetische Trennung entfernt. Die erste Selektionsrunde wurde dann eingeleitet durch 30 Minuten Inkubation der vorgewählten DNA-Bibliothek mit 50 pmol "Target"-Kügelchen bei Raumtemperatur. Nach dem Waschen und Entfernen ungebundener DNA mit 200 µl Selektionspuffer wurde gebundene DNA in 55 µl Wasser durch Erhitzen des Gemischs auf 80 °C für 3 Minuten eluiert. Anschließend wurde eluierte DNA mittels PCR unter Verwendung von 1 µM 5'-biotinyliertem Rückwärts-Primer (5'-GGGAGACAAGAATAAGCATG-3'), 1 µM Vorwärts-Primer (5'-GCCTGTTGTGAGCCTCCTAAC-3'), 1,5 mM MgCl₂, 200 µM dNTPs in 1x PCR-Puffer B und 0,05 U FIREPol® DNA-Polymerase pro µL (die beiden letzteren bezogen von Solis BioDyne) amplifiziert. Die Trennung von einsträngigen DNA-Aptameren von doppelsträngigen PCR-Produkten erfolgte mittels Streptavidin-beschichteter Magnetkügelchen. Daher wurden PCR-Produkte 1:2 in 2x B&W-Puffer (1 mM EDTA, 2 M NaCl in 10 mM Tris-HCl, pH 7,5) verdünnt, zu Streptavidin-beschichteten Magnetkügelchen zugegeben und anschließend zweimal mit 1x B&W-Puffer gewaschen. Nach Inkubation für 15 Minuten bei Raumtemperatur wurden die Überstände entfernt und die Kügelchen wurden in 150 mM NaOH resuspendiert, gefolgt von einer Inkubation für weitere 10 Minuten bei Raumtemperatur. Die vereinigten Überstände. die die Einzelstrang-DNA enthielten, wurden mit 100 mM HCl neutralisiert und als Ausgangspunkt für eine neue Selektionsrunde genutzt. Nach der Inkubation der DNA-Einzelstränge mit "Target"-Kügelchen wurde die Menge der Waschschritte von Runde zu Runde angehoben, wodurch die Stringenz anstieg.

### 1.4 Klonierung

Die Isolation von selektierten Aptameren wurde mittels molekularer TA-Klonierung durchgeführt. Dafür wurde der Vektor pUC19-T (pCR®2.1-TOPO®; Invitrogen) mit Xcml (Thermo Scientific) restringiert, um 3'-Thymidin-Überhänge zu erzeugen (Rong-liang H, Ting G, W, Ying W, Ye-Wei L, Cheng.Iong S. Construction of a pUC19-T vector Based on Xcm I. Agricultural Science & Technology. 2011;12(3):363-365; Marchuk D, Drumm M, Saulino A, Collins FS. Construction of T-vectors, a rapid and general system for direct cloning of unmodified PCR products. Nucleic Acids Res. 1991,19(5):1154). Aptamere wurden amplifiziert via PCR unter Verwendung von FIREPol®-DNA-Polymerase, die 5'-Adenosin-Überhänge erzeugt, und mit dem Vektor unter Verwendung von T4-DNA-Ligase (Thermo Scientific) ligiert. DNA von 50 erhaltenen Klonen wurde isoliert und sequenziert (GATC-Biotech).

### 2. Statistik

Für alle statistischen Analysen wurde GraphPad Prism 6 (GraphPad Software, Inc., La Jolla, CA 92037 USA) verwendet. Die Werte sind als Mittelwert ± Standardabweichung angegeben. Das "One-Site-Specific-binding"-Modell (GraphPad Prism 6) wurde für die Berechnung der Dissoziationskonstanten verwendet. Der zweiseitige ungepaarte t-Test wurde für Vergleiche zwischen zwei Gruppen verwendet. Signifikante Unterschiede zwischen zwei Mittel mit p <0,05 sind mit * gekennzeichnet, sehr signifikante Unterschiede (p < 0,01) mit ** und äußerst signifikante Unterschiede (p < 0,001) mit ***.

### 3. Filter-Retentions-Assays

Filter-Retentions-Assays (FRA) wurden verwendet, um die Dissoziationskonstanten der Aptamer-Selektin-Wechselwirkungen zu bestimmen. Hierzu wurde DNA mit [γ-³²P]-Adenosin-5'-triphosphat (Hartmann Analytic) unter Verwendung von T4-Polynukleotidkinase (Thermo Scientific) radioaktiv markiert und mittels Gelextraktion gefolgt von Isopropanol-Fällung gereinigt. Für Bindungstests wurden konstante Mengen radioaktiver DNA (<1 nM) mit steigenden Mengen entsprechender Proteine (0-1 µM) für 30 Minuten bei Raumtemperatur in Selektionspuffer inkubiert. Danach wurden die Protein-Aptamer-Komplexe durch eine Nitrozellulosemembran (Whatman) filtriert (Manifold I Dot-Blot-System, Whatman), die für 15 Minuten in 0,4 M KOH, für 5 Minuten in entmineralisiertem Wasser bei Raumtemperatur voräquilibriert und dann in Selektionspuffer gewaschen worden war. Nach Filtration wurde die Nitrocellulosemembran getrocknet und für 3 Stunden einem "Phosphorimaging"-Schirm (Bio-Rad) ausgesetzt. Zur Quantifizierung wurde das "One-site-specific-bonding"-Modell (Quantity One Software®) verwendet.

Die nachstehend wiedergegebenen (randomisierter Bereich unterstrichen) erfindungsgemäßen DNA-Aptamere, die im Folgenden als SDA1 (SDA = Selektin-DNA-Aptamer) und SDA2 bezeichnet werden, wurden mittels Filter-Retentions-Assay auf ihre Bindung an E- und P-Selektin untersucht (s. Figur 1):
SDA1 (SEQ ID NO: 1):
SDA2 (SEQ ID NO: 2):

Die DNA wurde wie oben beschrieben radiomarkiert, mit steigenden Mengen Protein (E-Selektin, P-Selektin, Streptavidin) inkubiert und durch eine Nitrocellulosemembran filtriert. Der Anteil gebundenen Aptamers wurde durch Autoradiographie ermittelt. Figur 1 zeigt, dass SDA1 (Fig. 1A) und SDA2 (Fig. 1B) sowohl an humanes E-Selektin als auch an humanes P-Selektin binden. Mittlere Dissoziationskonstanten (Kd) sind in Tabelle 1 wiedergegeben. Die DNA-Aptamere binden nicht an Streptavidin (Kontrolle), und Kontroll-DNA (eine 91 Nukleotide lange DNA, die sich von den erfindungsgemäßen DNA-Aptameren im randomisierten Bereich unterschied) bindet nicht an E- oder P-Selektin.

**Tabelle 1 Dissoziationskonstanten (K_{d}) für SDA1 und SDA2 gegenüber E- und P-Selektin**

| | E-Selektin | P-Selektin |
|---|---|---|
| SDA1 | 87 nM | 84 nM |
| SDA2 | 97 nM | 95 nM |

### 4. Nuklease-Resistenz/Stabilität

Das DNA-Aptamer wurde wie oben beschrieben mit [γ-³²P]-Adenosin-5'-triphosphat radioaktiv markiert und mit 100 µl Vollmedium bei 37 °C inkubiert. Nach bestimmten Zeiten (0-720 min) wurden 10 µl Probe in flüssigem Stickstoff eingefroren und anschließend via 10% denaturierender Gelelektrophorese analysiert.

Innerhalb der ersten 12 Stunden konnte zwar ein kontinuierlicher Abbau beobachtet werden. Selbst nach dieser Zeit waren aber noch mehr als 10% wirksame Aptamere detektierbar.

### 5. Interaktion der erfindungsgemäßen DNA-Aptamere mit Selektinen/Krebszellen

### 5.1 Zelllinien und Kultivierungsbedingungen

Die menschliche kolorektale Krebs-Zelllinie HT29 (European Cell Culture Collection, Katalognummer: 91072201) und die menschliche chronische eosinophile Leukämie-Zelllinie EOL-1 (DSMZ, ACC 386) wurden in RPMI-1640, versetzt mit 2 mM L-Glutamin, 10% fötalem Kälberserum (FCS), 100 µg/mLPenicillin und 100 µg/mL Streptomycin (letztere Reagenzien von PAA Laboratories GmbH, Pasching, Deutschland) bei 37 °C in einer befeuchteten Atmosphäre von 5 % CO₂ gehalten. Für alle Tests wurden HT29-Zellen bis 80 % Konfluenz kultiviert. EOL-1-Zellen wuchsen in Suspension. Humane primäre pulmonale mikrovaskuläre Endothelzellen (HPMECs) wurden von Promocell (PromoCell GmbH, D-69126 Heidelberg, Deutschland) erhalten und in Endothelzellwachstumsmedium MV, versetzt mit der vom Hersteller bereitgestellten entsprechenden Zusatzmischung und 100 µg/mL Penicillin und 100 µg/mL Streptomycin, kultiviert. Folgekulturen von HPMECs wurden mit einem speziellen Kit (PromoCell) gemäß den Anweisungen des Herstellers angelegt. Alle Experimente mit primären Zellen wurden während der ersten sechs Passagen durchgeführt.

### 5.2 Hemmung der statischen Tumorzelladhäsion an immobilisiertes menschliches E- und P-Selektin

Figur 2 zeigt die Aptamer-vermittelte Hemmung der statischen Interaktion zwischen Selektinen und HT29-Zellen. Die Aptamere wurden vor Inkubation mit HT29-Zellen mit PE-markiertem rekombinantem humanem (rh)-E- oder -P-Selektin präinkubiert. Die relative Bindung von HT29-Zellen wurde mittels Durchflusszytometrie in drei unabhängigen Experimenten bestimmt. SDA1 hemmte die Wechselwirkung von rhE-Selektin (Fig. 2A) und rhP-Selektin (Fig. 2C) mit HT29-Zellen selbst bei einer Konzentration von 1 µM (signifikant bei 2 µM). Ähnliches gilt für SDA2 (Fig. 2C), wobei SDA2 hier nur für P-Selektin untersucht wurde. Ein Überschuss eines Aptamer-Kompetitors zeigte keinen Einfluss auf die Bindung, was die Selektivität der Aptamer-Bindung zeigt (Fig. 2B, D).

### 5.3 Hemmung der dynamischen Tumorzelladhäsion an immobilisiertes menschliches E- und P-Selektin

Die Selektin-Ligand-Wechselwirkung und deren Aptamer-vermittelte Hemmung wurde unter Laminarströmungsbedingungen analysiert, die die endotheliale Scherspannung in postkapillären Venolen von metastatischen Organen wie beispielsweise Lungen repräsentiert (Sheikh S, Rainger GE, Gale Z, Rahman M, Nash GB. Exposure to fluid shear stress modulates the ability of endothelial cells to recruit neutrophils in response to tumor necrosis factor-alpha: a basis for local variations in vascular sensitivity to inflammation. Blood. 2003;102(8):2828-2834. DOI 10.1182/blood-2003-01-0080). Zu diesem Zweck wurden rhE- und rhP-Selektin immobilisiert auf IBIDI "ibiTreat" Mikroobjektträgern VI (ibidi GmbH, D-82152 Martinsried (München), Deutschland) in einer Endkonzentration von 0,02 mg/mL, verdünnt in DPBS enthaltend Ca²⁺ und Mg²⁺ (DPBS⁺; PAA) für 30 min bei 37 ° C. Als Kontrolle wurden Kammern parallel ebenfalls mit 0,02 mg/mL IgG-Fc (R&D Systems) beschichtet. Dann wurden die Objektträger mit 50 µl DPBS gewaschen und mit oder ohne (Positivkontrolle) Aptamer (0,15 mg Aptamer/mL DPBS⁺) für 30 Minuten bei Raumtemperatur inkubiert. IBIDI-Kapillaren wurden erneut mit 50 µl DPBS⁺ gewaschen und dann mit 1 x 10⁵ HT29-Tumorzellen pro ml bei einer laminaren Flussrate von 8,5 mL/h perfundiert (Sheikh S, Rainger GE, Gale Z, Rahman M, Nash GB. Exposure to fluid shear stress modulates the ability of endothelial cells to recruit neutrophils in response to tumor necrosis factor-alpha: a basis for local variations in vascular sensitivity to inflammation. Blood. 2003;102(8):2828-2834. DOI 10.1182/blood-2003-01-0080). Adhäsionsereignisse wurden aufgezeichnet und anschließend wie vorher beschrieben analysiert (Richter U, Schroder C, Wicklein D, et al. Adhesion of small cell lung cancer cells to E- and P-selectin under physiological flow conditions: implications for metastasis formation. Histochem Cell Biol. 2011;135(5):499-512. Prepublished on 2011/05/06 as DOI 10.1007/s00418-011-0804-4.)

Rh-Selektine wurden auf einer Laminarströmungs-Mikrokammer immobilisiert und die Kapillaren wurden dann mit HT29-Zellen perfundiert, und Adhäsions-Ereignisse wurden aufgezeichnet. Die Ergebnisse sind in Figur 3 dargestellt. Wenn keine beeinträchtigende Komponente vorhanden war, hefteten HT29-Zellen auf E-Selektin-beschichteten Kammern mit 11,89 ± 4,9 Ereignissen pro Minute an (Fig. 3A). Um die inhibitorische Wirkung von SDA zu untersuchen, wurden E-Selektin beschichtete Kammern mit SDA1 bzw. SDA2 vorinkubiert. Dadurch wurde die Anzahl der anheftenden HT29-Zellen deutlich reduziert. Im Falle von SDA2 reduzierten sich die Anheftungsereignisse beispielsweise auf 7,67 ± 3,9 pro Minute, entsprechend einer Reduktion von 35%. Kontroll-DNA zeigte keine hemmende Wirkung, so dass die HT29-Zelladhäsion nicht beeinträchtigt wurde (11,22 ± 5,3 Ereignisse/min). Als Negativkontrolle wurde IgG-Fc ohne Fusionspartner auf einer Mikrokammer immobilisiert, um eine unspezifische Bindung auszuschließen. Hier waren nur selten Adhäsionsereignisse zu beobachten (0,78 ± 0,8 Ereignisse/min , p <0,001 vs. E-Selektin).

Um zu untersuchen, ob SDA1/SDA2 auch die Zelladhäsion von EOL-1-Zellen an humanes P-Selektin hemmt, mit dem eine Kammer beschichtet wurde, wurden Versuche entsprechend den oben für E-Selektin beschriebenen vorgenommen. Im Laminarströmungstest ergab die ungehinderte EOL-1-Zelladhäsion an humanes P-Selektin 10,42 ± 3,7 Ereignisse pro Minute (3B). Dieser Wert wurde in Gegenwart von SDA1/SDA2 deutlich reduziert, im Falle von SDA2 beispielsweise auf 6,54 ± 2,2 Ereignisse pro Minute in (P <0,05 vs. P -Selektin), entsprechend einer Reduktion auf 60 %. Bei Inkubation mit Kontroll-DNA blieb die Adhäsion der EOL-1-Zellen an P-Selektin mit 13,00 ± 3,7 Ereignisse pro Minute unverändert.

### 5.4 Hemmung der dynamischen Tumorzelladhäsion an menschliches Lungenendothel (Zell-Zell-Interaktion)

Um das hemmende Potenzial der erfindungsgemäßen Aptamere auf scherfeste Tumorzelladhäsion gegenüber menschlichem Lungenendothel zu bestimmen, wurden IBIDI ibiTreat-Mikroobjekttrager VI mit konfluenten HPMEC-Monoschichten beschichtet, die unbehandelt blieben oder für 4 h vor dem Strömungs-Adhäsions-Assay (10 ng/mL) mit rekombinantem humanem (rh) TNFα (PeproTech GmbH, Hamburg, Deutschland) stimuliert wurden, da nicht-stimulierte HPMECs E-Selektin nicht auf ihrer Oberfläche präsentieren. Die stimulierten HPMECs wurden mit 0,15 mg Aptamer pro mL Medium für 30 Minuten bei 37 ° C inkubiert und dann mit 1 x 10⁵ HT29-Tumorzellen pro ml wie oben beschrieben perfundiert (Kontrolle ohne Aptamer).

Aufgrund der oben beschriebenen Stimulierung erhöhte sich die Zahl der an HPMECs anhaftenden HT29-Zellen von 1,50 ± 1,3 Zellen pro Minute (-rhTNFα, P <0,01 vs stimulierten HPMEC) auf 23,17 ± 12,7 Zellen pro Minute (+rhTNFα). Um den Einfluss der Aptamere auf diese Zell-Zell-Interaktion zu untersuchen, wurden rhTNFα-stimulierte HPMECs entweder mit einem erfindungsgemäßen Aptamer oder mit Kontroll-DNA inkubiert. Der folgende Laminarströmungstest mit HT29-Zellen zeigte, dass die Aptamere die HT29-Adhäsion an E-Selektin präsentierende HPMECs signifikant auf bis zu 45 % reduzierte (10,50 ± 2,1 Ereignisse/min für SDA2, P <0,05 vs stimuliert HPMECs). Im Gegensatz dazu zeigte Kontroll-DNA keine signifikante Wirkung (19,67 ± 7,7 Ereignisse/min, P = 0,58; Fig. 3C).

### 6. Stabilität in Plasma

Die Stabilität von SDA2 und einer "Scramble"-Variante (SDA2-SCR, SEQ ID NO: 5) davon, d.h. einer Variante mit gleicher Basenzusammensetzung, jedoch anderer Basenreihenfolge, wurde in murinem Plasma mittels denaturierender Polyacrylamid-Gelelektrophorese untersucht. Die radioaktiv markierten Nukleinsäuren wurden für definierte Zeiträume bei 37°C in murinem Plasma inkubiert, in flüssigem Stickstoff zwischengelagert und anschließend mittels PAGE analysiert. Das erfindungsgemäße Aptamer SDA2 ist, wie aus Figur 4 ersichtlich, über 24 Stunden im murinen Plasma stabil. Die als Kontrolle ebenfalls mit untersuchte Scramble-Variante des Aptamers (SDA2-SCR) ist demgegenüber nach 24 Stunden nur noch zu ca. 30% in voller Länge vorhanden.
SDA2-SCR (SEQ ID NO: 5)

Sequenzprotokoll - freier Text und Übersetzung englischer Ausdrücke
Artificial Sequence = Künstliche Sequenz
Unassigned DNA = Nicht zugeordnete DNA
DNA aptamer = DNA-Aptamer
Forward Primer = Vorwärts-Primer
Reverse primer = Rückwärts-Primer
Scramble variant = "Scramble"-Variante

## Patentansprüche

1. DNA-Aptamer, das menschliches E-Selektin und P-Selektin spezifisch bindet und
a) eine Sequenz mit den Nukleotiden 22-71 gemäß SEQ ID NO: 1 oder SEQ ID NO: 2, oder
b) eine Sequenz mit mindestens 75%, 80%, 85%, 90%, 95%, 96%, 97%, 98% oder 99% Identität zu einer Sequenz mit den Nukleotiden 22-71 gemäß SEQ ID NO: 1 oder SEQ ID NO: 2, oder
c) eine Sequenz gemäß a) oder b) mit mindestens einem modifizierten Nukleotid umfasst, wobei das mindestens eine modifizierte Nukleotid ein 2'-Desoxy, 2'-Halogenid-, 2'-Amino-, 2'-O-Methyl- oder 2'-Methoxyethyl-Nukleotid ist.

2. DNA-Aptamer nach Anspruch 1, **dadurch gekennzeichnet, dass** das DNA-Aptamer eine der Sequenzen gemäß SEQ ID NO: 1 oder SEQ ID NO: 2 hat oder umfasst.

3. DNA-Aptamer nach einem der vorhergehenden Ansprüche, **dadurch gekennzeichnet, dass** das DNA-Aptamer PEGyliert ist.

4. Nukleinsäure, umfassend oder bestehend aus einem Oligomer eines DNA-Aptamers nach einem der Ansprüche 1 bis 3.

5. Nukleinsäure nach Anspruch 4, **dadurch gekennzeichnet, dass** das Oligomer zwei, drei, vier, fünf, sechs, sieben, acht, neun oder zehn Wiederholungseinheiten aufweist.

6. Nukleinsäure nach Anspruch 4 oder 5, **dadurch gekennzeichnet, dass** das Oligomer ein Mischoligomer aus mindestens zwei verschiedenen Wiederholungseinheiten ist.

7. DNA-Aptamer nach einem der Ansprüche 1 bis 3 oder Nukleinsäure nach einem der Ansprüche 4 bis 6 zur Verwendung als Medikament.

8. DNA-Aptamer nach einem der Ansprüche 1 bis 3 oder Nukleinsäure nach einem der Ansprüche 4 bis 6 zur Verwendung als Medikament gegen Entzündung und/oder Krebs und/oder zur Förderung der Geweberegeneration.

9. Arzneimittel, umfassend ein DNA-Aptamer nach einem der Ansprüche 1 bis 3 oder eine Nukleinsäure nach einem der Ansprüche 4 bis 6.

10. Verwendung eines DNA-Aptamers nach einem der Ansprüche 1 bis 3 oder einer Nukleinsäure nach einem der Ansprüche 4 bis 6 in vitro zum Nachweis und/oder zur Darstellung und/oder zur Isolierung von E-Selektin oder P-Selektin oder von Zellen, die E-Selektin oder P-Selektin präsentieren.

## Claims

1. A DNA aptamer that specifically binds human E-selectin and P-selectin and comprises
a) a sequence with the nucleotides 22-71 according to SEQ ID NO: 1 or SEQ ID NO: 2, or
b) a sequence having at least 75%, 80%, 85%, 90%, 95%, 96%, 97%, 98% or 99% identity to a sequence with the nucleotides 22-71 according to SEQ ID NO: 1 or SEQ ID NO: 2, or
c) a sequence according to a) or b) having at least one modified nucleotide, wherein the at least one modified nucleotide is a 2'-deoxy, 2'-halide, 2'-amino, 2'-O-methyl or 2'-methoxyethyl nucleotide.

2. A DNA aptamer according to claim 1, **characterized in that** the DNA aptamer has or comprises one of the sequences according to SEQ ID NO: 1 or SEQ ID NO: 2.

3. A DNA aptamer according to one of the preceding claims, **characterized in that** the DNA aptamer is PEGylated.

4. A Nucleic acid, comprising or consisting of an oligomer of a DNA aptamer according to one of claims 1 to 3.

5. A Nucleic acid according to claim 4, **characterized in that** the oligomer has two, three, four, five, six, seven, eight, nine or ten repeat units.

6. A Nucleic acid according to claim 4 or 5, **characterized in that** the oligomer is a mixed oligomer of at least two different repeat units.

7. A DNA aptamer according to one of claims 1 to 3 or a nucleic acid according to one of claims 4 to 6 for use as a medicament.

8. A DNA-Aptamer according to one of claims 1 to 3 or a nucleic acid according to one of claims 4 to 6 for use as a medicament against inflammation and/or cancer and/or for promoting tissue regeneration.

9. A medicament comprising a DNA aptamer according to one of claims 1 to 3 or a nucleic acid according to one of claims 4 to 6.

10. Use of a DNA aptamer according to one of claims 1 to 3 or a nucleic acid according to one of claims 4 to 6 in vitro for the detection and/or for the imaging and/or for the isolation of E-selectin or P-selectin or of cells presenting E-selectin or P-selectin.

## Revendications

1. Aptamère ADN, liant la E-sélectine et la P-sélectine humaines et
a) comprenant une séquence avec les nucléotides 22-71 selon la SEQ ID NO: 1 ou la SEQ ID NO: 2, ou
b) une séquence avec au moins 75%, 80%, 85%, 90%, 95%, 96%, 97%, 98% ou 99% d'identité avec une séquence avec les nucléotides 22-71 selon la SEQ ID NO: 1 ou la SEQ ID NO: 2, ou
c) une séquence selon a) ou b) avec au moins un nucléotide modifié, l'au moins un nucléotide modifié étant un 2'-désoxy, 2'-halogénure-, 2'-amino-, 2'-O-méthyl- ou 2'-méthoxyéthyl-nucléotide

2. Aptamère ADN selon la revendication 1, **caractérisé en ce que** l'aptamère ADN a ou comprend l'une des séquences selon la SEQ ID NO: 1 ou la SEQ ID NO: 2.

3. Aptamère ADN selon l'une quelconque des revendications précédentes, **caractérisé en ce que** l'aptamère ADN est pégylé.

4. Acide nucléique, comprenant un ou constitué d'un oligomère d'un aptamère ADN selon l'une quelconque des revendications 1 à 3.

5. Acide nucléique selon la revendication 4, **caractérisé en ce que** l'oligomère présente deux, trois, quatre, cinq, six, sept, huit, neuf ou dix unités de répétition.

6. Acide nucléique selon la revendication 4 ou 5, **caractérisé en ce que** l'oligomère est un oligomère mixte d'au moins deux différentes unités de répétition.

7. Aptamère ADN selon l'une quelconque des revendications 1 à 3 ou acide nucléique selon l'une quelconque des revendications 4 à 6, destiné à être utilisé en tant que médicament.

8. Aptamère ADN selon l'une quelconque des revendications 1 à 3 ou acide nucléique selon l'une quelconque des revendications 4 à 6, destiné à être utilisé en tant que médicament contre les inflammations et/ou le cancer et/ou pour stimuler la régénération tissulaire.

9. Médicament, comprenant un aptamère ADN selon l'une quelconque des revendications 1 à 3 ou un acide nucléique selon l'une quelconque des revendications 4 à 6.

10. Utilisation in-vitro d'un aptamère ADN selon l'une quelconque des revendications 1 à 3 ou d'un acide nucléique selon l'une quelconque des revendications 4 à 6 pour déceler et/ou pour représenter et/ou pour isoler de la E-sélectine et/ou de la P-sélectine ou des cellules qui présentent de l'E-sélectine ou de la P-sélectine.
